Europäisches Patentamt

European Patent Office

Office européen des brevets

(19)

(11) Publication number: 0 204 413

A1

# (12) EUROPEAN PATENT APPLICATION

(21) Application number: 86302937.7

(22) Date of filing: 18.04.86

(51) Int. Cl.⁴: C 07 C 19/00

(30) Priority: 26.04.85 GB 8510668

(43) Date of publication of application:
10.12.86 Bulletin 86/50

(84) Designated Contracting States:
DE FR GB

(71) Applicant: IMPERIAL CHEMICAL INDUSTRIES PLC
Imperial Chemical House Millbank
London SW1P 3JF(GB)

(72) Inventor: Mills, John Frederick David
Fieldings Smithy Lane
Mouldsworth Chester CH3 8AR(GB)

(72) Inventor: Richardson, Michael Thomas
Keepers Cottage Townfield Lane
Mollington NR Chester(GB)

(72) Inventor: Madeley, John Robert
Beverly 19 Greenover Road
Brixham Devon TQ5 9LY(GB)

(74) Representative: Oldroyd, Alan et al,
Imperial Chemical Industries PLC Legal Department:
Patents PO Box 6 Bessemer Road
Welwyn Garden City Herts, AL7 1HD(GB)

(54) Halogenated hydrocarbons.

(57) A chlorinated paraffins composition of low or zero toxicity comprising a chlorinated mixture of a $C_{12}$ paraffin and one or more paraffins of chain length greater than 12 carbon atoms and containing from 20 to 70% by weight chlorine based on the chlorinated paraffins; preferably a chlorinated mixture of $C_{12}$, $C_{13}$, $C_{14}$ and optionally $C_{15}$ paraffins.

EP 0 204 413 A1

0204413

QM 33454|EP

HALOGENATED HYDROCARBONS

This invention relates to halogenated hydrocarbons and particularly to chlorinated mixed paraffinic hydrocarbons.

Chlorinated straight-chain paraffinic hydrocarbons, usually referred to simply as chlorinated paraffins, are well known materials which are used extensively in industry in a wide variety of applications, for example as plasticizers for vinyl chloride and other polymers and copolymers and as additives in coatings, adhesives and lubricants. They are produced by chlorinating straight-chain paraffin oils and waxes containing carbon chains of length in the range from about 10 to about 30 carbon atoms to a chlorine content of greater than about 20 percent by weight, typically from about 40 to about 70% by weight.

Although chlorinated single paraffins are known, the common commercially-available products comprise mixtures obtained by chlorinating mixed paraffins of different carbon chain length. The mixed paraffin fractions or "cuts" used in the production of the common products are a nominal $C_{10}$-$C_{13}$ fraction, a nominal $C_{14}$-$C_{17}$ fraction and a nominal $C_{20}$-$C_{30}$ fraction. In practice, each nominal fraction or 'cut' may contain small amounts, for example a few percent, of paraffins of chain length outside the specified range; thus for example the nominal $C_{10}$-$C_{13}$ fraction may contain small proportions of one or more of a $C_8$, $C_9$, $C_{14}$ and $C_{15}$ paraffin.

The chlorinated paraffins have always, at least until recently, been regarded as materials of a very low order of toxicity whose use in large amounts posed minimal or no environmental problems. This still appears to be the case in respect of long-chain chlorinated paraffins such as the common $C_{14}$-$C_{17}$ products and $C_{20}$-$C_{30}$ products. In recent years, however, laboratory experiments under rigorous conditions of continuous exposure of specimens to chlorinated paraffins in water for long periods, for example up to 60 days, have revealed that the short chain chlorinated paraffins such as the $C_{10}$-$C_{13}$ products can be toxic to certain aquatic species. For example, the $C_{10}$-$C_{13}$ products exhibit evidence of toxicity under conditions of continuous exposure towards rainbow trout at concentrations greater than about 30 micrograms per litre and towards seawater mussels at concentrations greater than about 10 to 12 micrograms per litre. The susceptibility of test species to the short chain chlorinated paraffins has been shown to depend to some extent upon the chlorine content of the products, being in general greater the higher the chlorine content, but toxicity has been observed in respect of the common $C_{10}$-$C_{13}$ product containing about 50-60 percent by weight chlorine. Evidence has also been reported of bioaccumulation of these short-chain, chlorinated paraffins in several aquatic species such as trout, bleak and mussels.

Although the $C_{10}$-$C_{13}$ chlorinated paraffins exhibit toxic effects only under rigorous experimental conditions, it is nevertheless desirable, especially in view of the observed bioaccumulation in aquatic species, to provide alternative products which do not exhibit such effects.

We have now found, using rainbow trout as the principal test species, that the observed toxicity of chlorinated short-chain paraffins and notably of the common $C_{10}$-$C_{13}$ products, is not exhibited at concentrations up to 1000 micrograms per litre by chlorinated mixed paraffins of chain length $C_{12}$ and greater providing that the mixture is free or essentially free from paraffins of chain length $C_{11}$ and less.

According to the present invention there is provided a chlorinated paraffins composition of chlorine content from 20-70% by weight based on the chlorinated paraffins and comprising a chlorinated mixture of a $C_{12}$ paraffin and one or more paraffins of chain length greater than 12 carbon atoms. A preferred embodiment of the invention resides in a chlorinated mixed-paraffin composition comprising chlorinated paraffins of chain lengths 12, 13, 14 and optionally 15 carbon atoms.

The compositions are preferably free or essentially free from chlorinated paraffins of chain length below 12 carbon atoms and in particular preferably contain less than 3% and especially less than 1% by weight based on the total chlorinated paraffins of chlorinated paraffins of chain length 11 or less carbon atoms. It is especially preferred that the content of chlorinated paraffins of 11 or less carbon atoms be less than 0.5% by weight of the total weight of chlorinated paraffins.

The chlorinated mixed-paraffins provided according to the invention may be produced by chlorination of a suitable mixed-paraffin feedstock to the desired chlorine content in known manner. Any of the known methods for the production of chlorinated paraffins may be employed, these methods generally comprising passing chlorine gas into the liquid paraffins at a temperature above about 80°C.

The chlorinated paraffin compositions of the invention may contain any of the additives such as stabilizers and modifying agents normally incorporated in chlorinated paraffin compositions and they may be used in any of the applications in which chlorinated paraffins are commonly used. Compositions containing relatively short-chain chlorinated paraffins and in particular compositions containing chlorinated $C_{12}$, $C_{13}$, $C_{14}$ and $C_{15}$ paraffins can be used in applications where the common $C_{10}-C_{13}$ product is deemed to be unacceptable owing to its observed toxicity.

CLAIMS

1. A chlorinated paraffins composition of chlorine content 20-70% by weight based on the chlorinated paraffins and comprising a chlorinated mixture of a $C_{12}$ paraffin and one or more paraffins of chain length greater than 12 carbon atoms.

2. A composition as claimed in claim 1 comprising chlorinated paraffins of chain lengths 12, 13, 14 and 15 carbon atoms.

3. A composition as claimed in claim 1 comprising chlorinated paraffins of chain lengths 12, 13 and 14 carbon atoms.

4. A composition as claimed in claim 1, 2 or 3 which contains less than 3% by weight based on the total chlorinated paraffins of chlorinated paraffins of chain length 11 or less carbon atoms.

5. A composition as claimed in claim 4 wherein the content of chlorinated paraffins of chain length 11 or less carbon atoms is less than 1% by weight based on the total weight of chlorinated paraffins.

6. A composition as claimed in any of claims 1 to 5 which contains one or more of the known additives normally incorporated in chlorinated paraffin compositions.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMOSPHERE, no. 2, 1975, pages 83-88, Pergamon Press, Oxford, GB; E.S. LAHANIATIS et al.: "Beiträge zur Ökologischen Chemie XCIV zur Analytik und Photochemie hochsiedender polychlorierter Paraffingemische" * Whole document * | 1-5 | C 07 C   19/00 |
| A | DE-C- 871 888  (DORTMUNDER PARAFFIN-WERKE) | | |

TECHNICAL FIELDS
SEARCHED (Int. Cl.4)

C 07 C   19/00

The present search report has been drawn up for all claims

| Place of search | Date of completion of the search | Examiner |
|---|---|---|
| THE HAGUE | 20-08-1986 | VAN GEYT J.J.A. |